Europäisches Patentamt

European Patent Office

Office européen des brevets

(11) Numéro de publication : **0 458 674 B1**

(12) **FASCICULE DE BREVET EUROPEEN**

(45) Date de publication du fascicule du brevet :
06.10.93 Bulletin 93/40

(51) Int. Cl.⁵ : **B01J 29/32,** C07C 2/00,
C10G 31/00, C07C 15/00

(21) Numéro de dépôt : **91401224.0**

(22) Date de dépôt : **10.05.91**

(54) **Catalyseur zéolitique et son utilisation dans l'aromatisation d'hydrocarbures contenant 2 à 4 atomes de carbone.**

(30) Priorité : **23.05.90 FR 9006557**

(43) Date de publication de la demande :
**27.11.91 Bulletin 91/48**

(45) Mention de la délivrance du brevet :
**06.10.93 Bulletin 93/40**

(84) Etats contractants désignés :
**DE ES GB IT NL**

(56) Documents cités :
**EP-A- 0 228 267**
**EP-A- 0 361 424**

(73) Titulaire : **INSTITUT FRANCAIS DU PETROLE**
**4, Avenue de Bois Préau**
**F-92502 Rueil-Malmaison (FR)**

(72) Inventeur : **Bournonville, Jean-Paul**
**43, rue des Groues Vauréal**
**F-95000 Cergy Pontoise (FR)**
Inventeur : **Raatz, Francis**
**25, rue de la Carrière**
**F-57500 Saint-Avold (FR)**
Inventeur : **Juguin, Bernard**
**46, avenue du Stade**
**F-92500 Rueil Malmaison (FR)**

EP 0 458 674 B1

Jouve, 18, rue Saint-Denis, 75001 PARIS

## Description

La présente invention concerne un catalyseur composite comprenant d'une part une zéolithe de structure MFI contenant du silicium, de l'aluminium et d'autre part un métal de la famille du platine déposé sur un support oxyde réfractaire sur lequel sont ajoutés au moins un additif métallique choisi dans le groupe constitué par l'étain, le germanium, l'indium et le plomb et au moins un métal alcalin ou un métal alcalino terreux.

En outre, l'invention concerne l'utilisation de ce catalyseur composite dans les réactions d'aromatisation des hydrocarbures comprenant entre 2 et 4 atomes de carbone par molécule.

La réaction d'aromatisation du propane et du butane en présence d'un catalyseur contenant de la zéolithe ZSM5 (ou MFI) et du gallium a été découvert et breveté par la société British Petroleum (US-A 4175057 et 4180689). D'autres sociétés ou organismes ont depuis cette date déposé des brevets relatifs à des modifications du solide (US-A-4795844, Brevet Européen EP-B-0252705) et/ou à des changements de charge : coupe $C_2$-$C_{12}$ (Brevet Européen EP-B-0252705), éthane et éthylène (Brevet Européen EP-B-0050021 et US-A-4350835). Le mode d'introduction du gallium a fait également l'objet de prise de brevet (Brevets Européens EP-B-0120018 et 0184927).

L'ajout d'un autre métal au système Ga-MFI a été aussi envisagé pour améliorer les sélectivités en aromatiques et pour réduire la quantité de coke sur le catalyseur.

Ainsi l'imprégnation d'un catalyseur Ga/MFI par du rhénium, associé au platine ou au palladium permet d'améliorer de façon significative la sélectivité de production d'aromatiques (US-A-4766265). D'autres brevets revendiquent l'addition de platine et de palladium au catalyseur Ga-MFI (US-A-4407728, Brevets Européens EP-B-0215579, 0216491, 0224162, 0228267 et Brevets Japonais 61268634, 62081329, 612277636).

L'addition de platine à la zéolithe MFI permet d'améliorer la conversion du propane (T. INUI, F. OKAZUNI, Y. MAKINO, Chem. Express 1 (1), 53-56, 1985). Cependant la sélectivité de production de méthane et d'éthane est sensiblement augmentée. L'adjonction de rhénium au platine accentue encore la production de méthane et d'éthane par hydrogénolyse (S. ENGELS et coll, Catalysis Today, 3, 437-443, 1988). L'ajout de cuivre (P. MERIAUDEAU et coll, Zeolites : Facts, Figures, Futur, 1423-29, 1989) et de chrome (E.S SHPIRO et coll International Symposium on Zeolites as catalysts, Sorbents and Detergent builders, Würzburg (RFA), p. 73, 1988) diminue la production de méthane, mais la sélectivité en aromatiques reste inférieure aux systèmes Ga/MFI. Par ailleurs, très récemment l'addition de soufre à un catalyseur Pt/MFI permet d'en améliorer sensiblement la sélectivité de production d'aromatiques à partir de paraffines contenant de 6 à 12 atomes de carbones (US-A-4835336).

On a découvert que des performances améliorées par rapport à ce qui est connu dans l'art antérieur peuvent être obtenues en aromatisation des hydrocarbures légers en utilisant un nouveau catalyseur composite.

Ce catalyseur comprend une zéolithe de structure MFI d'une part, et d'autre part un support ou une matrice généralement amorphe sur lequel est déposé un métal noble de la famille du platine et, au moins un métal additionnel choisi dans le groupe constitué par l'étain, le germanium, le plomb, et l'indium, le dit support contenant également au moins un métal alcalin ou au moins un métal alcalino terreux choisi dans le groupe constitué par le lithium, le sodium, le potassium, le rubidium, le césium, le baryum, le calcium, le baryllium, le magnésium et le strontium.

La zéolithe de structure MFI constituant une partie du catalyseur de la présente invention, peut être préparée par toutes les techniques connues dans l'art antérieur. La synthèse de la-dite zéolithe MFI peut être réalisée en milieu classique OH⁻, en présence ou non de structurants organiques et/ou d'alcool. La synthèse de la zéolithe MFI en milieu OH⁻ selon les techniques connues dans l'art antérieur est largement décrite dans le document suivant : Synthesis of High Silica Zeolites, P. Jacobs and J. Martens, Studies in Surface Science and Catalysis, Volume 33, Elsevier editor, 1987. La zéolithe MFI peut également être synthétisée dans des milieux moins classiques comme par exemple le milieu fluorure (Brevet Européen EP-A-172068 de C.F.R).

Après synthèse, la zéolithe MFI est transformée en une forme hydrogène par élimination totale ou partielle des composés organiques et/ou des cations alcalins ou alcalino-terreux qu'elle contient éventuellement après la synthèse. Toutes les techniques connues dans l'art antérieur peuvent être utilisées pour passer à la forme hydrogène, comme par exemple les calcinations sous atmosphère oxydante ou non, les échanges ioniques suivis ou non de calcination, les traitements chimiques divers etc.

Toutes les zéolithes MFI synthétisées dans le système Si-Al conviennent pour la présente invention. Cependant leur rapport Si/Al sera supérieur à 7, de préférence supérieur à 25 et de préférence compris entre 40 et 200.

Les supports des métaux ajoutés à la zéolithe sont généralement choisis parmi les oxydes des métaux des groupes II, III et/ou IV de la classification périodique des éléments, tels que par exemple, les oxydes de magnésium, d'aluminium, de titane, de zirconium, de thorium ou de silicium, pris seuls ou en mélange entre eux ou avec des oxydes d'autres éléments de la classification périodique, tels que par exemple le bore. On

2

peut aussi utiliser du charbon.

Le support préféré est l'alumine ; la surface spécifique de l'alumine peut avantageusement être comprise entre 50 et 600 m² par gramme, de préférence entre 150 et 400 m²/g.

Le catalyseur composite de la présente invention peut être préparé suivant deux voies dont le principe est donné ci-après.

Première voie : Mélange de la zéolithe MFI avec le support ; ce mélange peut être réalisé entre deux poudres, entre les deux solides mis en forme, entre une poudre et un des solides mis en forme. On peut également mettre en forme conjointement les deux solides par toutes les techniques connues dans l'art antérieur : pastillage, extrusion, dragéification, coagulation en goutte, séchage par atomisation. Lors de ces opérations de mise en forme, on pourra si nécessaire ajouter un additif de mise en forme (silice etc). Après mélange et/ou mise en forme, on procède au dépôt des divers agents actifs sur le support (en présence donc de la zéolithe).

Deuxième voie : on dépose préalablement les agents actifs sur le support, que l'on mélange ou que l'on met en forme avec la zéolithe MFI dans les mêmes conditions que précédemment. Dans une variante, la zéolithe pourra être introduite dans le catalyseur composite à l'une quelconque des étapes de dépôt des agents actifs sur le support.

La méthode préférée de préparation consiste à déposer les agents actifs sur le support puis à introduire la zéolithe dans le catalyseur final par mise en forme des deux poudres. La mise en forme sera de préférence réalisée après un broyage micronique, qui pourra être réalisé en utilisant la technique de broyage humide.

Le catalyseur composite contient entre 1 et 99 % en poids de zéolithe, le complément à 100 % étant constitué par le support chargé en différents agents actifs. La proportion respective de zéolithe et de support varie dans une large gamme, car elle dépend d'une part du rapport Si/Al de la zéolithe et d'autre part de la teneur en agents actifs du support.

La partie du catalyseur composite comprenant du métal noble est généralement préparée selon des méthodes classiques consistant à imprégner le support au moyen de solutions de composés des métaux que l'on désire introduire. On utilise soit une solution commune de ces métaux, soit des solutions distinctes pour le métal de la famille du platine et pour le ou les métaux additionnels. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

Le platine (et éventuellement un autre métal noble du groupe du platine) peut être incorporé dans le support par imprégnation de ce support à l'aide d'une solution adéquate aqueuse ou non renfermant un sel ou un composé du métal noble. Le platine est généralement introduit dans le support sous forme d'acide chloroplatinique mais peuvent également être utilisés des composés tels que le chloroplatinate d'ammonium, le dichlorure de platine dicarbonyle, l'acide hexahydroxyplatinique, le chlorure de palladium, le nitrate de palladium.

L'élément choisi dans le groupe constitué de l'étain, du germanium, du plomb et de l'indium peut être introduit par l'intermédiaire de composés tels que par exemple les chlorures, les bromures et le nitrate d'étain, les halogénures, nitrate, acétate et carbonate de plomb, le chlorure et l'oxalate de germanium, le nitrate et le chlorure d'indium.

L'élément choisi dans le groupe constitué par les alcalins et alcalino-terreux peut être introduit par l'intermédiaire de composés tels que les halogénures, les nitrates, les carbonates, cyanures, oxalates.

Un procédé de fabrication comprend les étapes suivantes :

a) introduction sur le support d'au moins un élément choisi dans le groupe constitué par les alcalins et les alcalino-terreux.

b) Calcination du produit obtenu à l'étape a).

c) Introduction sur le support d'au moins un métal noble de la famille du platine, sous la forme d'au moins un composé halogéné dudit métal.

d) Calcination du produit obtenu à l'étape c).

e) Introduction sur le produit obtenu à l'étape b) d'au moins un métal additionnel sous la forme d'au moins un composé organométallique dudit métal M.

Parmi les composés du ou des métaux de la famille du platine que l'on emploie dans la présente invention, on peut citer à titre d'exemple les complexes ammoniés.

On citera en particulier dans le cas du platine les sels de platine IV hexamines de formule $(Pt(NH_3)_6)X_4$ où X est un atome d'halogène choisi dans le groupe formé par le fluor, le brome et l'iode et de préférence X est un atome de chlore.

Les sels de platine IV halogénopentammines de formule $(Pt\ X(NH_3)_5)X_3$.

Les platine IV tétrahalogénodiammines de formule $Pt\ X_4(NH_3)_2$ ou X a la signification donnée ci-dessus. Les complexes du platine avec les halogènes-polycétones et les composés halogénés polycétoniques de formule $H(Pt(aca)_2X)$ dans lesquels X à la signification donnée ci-dessus et aca représente le reste de formule

3

$C_5H_7O_2$ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables on peut citer les hydrocarbures paraffiniques, naphténiques ou aromatiques et les composés organiques halogénés ayant par exemple de 1 à 12 atomes de carbone dans leur molécule. On citera en particulier le n-heptane, le méthylcyclohexane, le toluène et le chloroforme. On peut aussi utiliser des mélanges de solvants.

Les supports sont les supports classiques tels que ceux définis ci-dessus et contenant déjà par exemple un alcalin ou un alcalino terreux.

Après introduction du métal noble de la famille du platine, le produit obtenu est éventuellement séché puis calciné de préférence à une température d'environ 400 à 1000°C.

Après cette calcination on procède à l'introduction du ou des métaux additionnels, éventuellement avant d'introduire ledit métal M on procède à une réduction à l'hydrogène à haute température, par exemple de 300 à 500°C. Cette réduction peut consister par exemple en une montée lente de la température sous courant d'hydrogène jusqu'à la température maximale de réduction, comprise par exemple entre 300 et 500°C et de préférence entre 350 et 450°C suivie d'un maintien sous hydrogène pendant 1 à 6 heures à cette température.

Le métal additionnel M peut être introduit avant ou après l'introduction du métal noble. S'il est introduit avant le métal noble, le composé utilisé sera choisi dans le groupe constitué par les halogénures, nitrates, acétates, carbonates, oxalates du métal additionnel. L'introduction sera avantageusement effectuée en solution aqueuse. Dans ce cas, avant de procéder à l'introduction du métal noble, on procède à une calcination sous air à une température comprise entre 400 et 1000 degrés centigrades.

Le métal additionnel M peut être introduit après l'introduction du métal noble sous la forme d'au moins un composé organique choisi dans le groupe formé par les complexes, en particulier les complexes polycétoniques, des métaux M et les hydrocarbylmétaux tels que les alkyles, les cycloalkyles, les aryles, les alkylaryles et les arylalkyles métaux.

L'introduction du métal M est avantageusement effectuée à l'aide d'une solution dans un solvant organique du composé organométallique dudit métal M. On peut également employer des composés organohalogénés des métaux M. Comme composés de métaux M on citera en particulier le tétrabutyl-étain, le tétraméthyl-étain, le tétrapropyl-germanium, le tétraéthyl-plomb, l'acétylacétonate d'indium, le triphénylindium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut utiliser des mélanges des solvants définis ci-dessus.

Cette méthode d'introduction du métal M a déjà été décrite dans le brevet US-A-4548918. Mais la combinaison de la méthode d'introduction du métal de la famille du platine et de la méthode d'introduction du métal M engendre une synergie particulière.

La partie du catalyseur composite, contenant le métal noble renferme en poids par rapport au support (a) environ 0,01 à 2 % et plus particulièrement environ 0,1 à 0,5 % d'au moins un métal noble de la famille du platine, (b) environ 0,005 à 0,60 %, et de préférence 0,01 à 0,50 d'étain ou 0,005 à 0,70 % de préférence environ 0,01 à 0,6 % et plus particulièrement 0,02 à 0,50 % d'au moins un métal choisi dans le groupe constitué par le germanium, le plomb et l'indium, (c) environ 0,01 à 2 % et plus particulièrement environ 0,1 à 0,6 % d'au moins un métal choisi dans le groupe constitué par les alcalins et les alcalino-terreux et de préférence le lithium et le potassium et leurs mélanges.

Lorsqu'il y a au moins deux métaux de la famille étain, germanium, plomb et indium, la teneur globale en métaux de cette famille est d'environ 0,02 à 1,20 % et de préférence 0,02 à 1,0 % et plus particulièrement 0,03 à 0,80 %.

Dans le procédé de l'invention, à l'issue de la préparation de la partie du catalyseur composite contenant le métal noble, celui-ci est généralement calciné entre 450 et 1000°C mais le catalyseur, à l'issue de la calcination peut subir avantageusement un traitement d'activation sous hydrogène à haute température, par exemple 300-500°C, afin d'obtenir une phase métallique plus active. La procédure de ce traitement sous hydrogène consiste par exemple en une montée lente de la température sous courant d'hydrogène jusqu'à la température maximale de réduction, comprise par exemple 300 et 500°C et de préférence entre 350 et 450°C, suivie d'un maintien pendant 1 à 6 heures à cette température.

Ce type de préparation du catalyseur conduit à un solide dans lequel les métaux sont répartis de façon homogène dans tout le volume du grain de catalyseur, et sont dans un état métallique après le traitement de réduction sous balayage d'hydrogène entre 300 et 500°C et maintien pendant 1 à 6 heures sous hydrogène à la température finale choisie.

A titre d'exemple une méthode plus particulière de préparation des catalyseurs consiste à effectuer les étapes suivantes :

(a) on imprègne un support d'alumine avec une solution aqueuse de nitrate de lithium,

(b) on sèche le produit obtenu à l'étape (a),

(c) on calcine le produit obtenu à l'étape (b),

(d) on imprègne le produit obtenu à l'étape (c) par une solution aqueuse d'un composé d'un métal choisi dans le groupe constitué par l'étain, le germanium, l'indium et le plomb,

(e) on sèche le produit obtenu à l'étape (d),

(f) on calcine le produit obtenu à l'étape (e),

(g) on imprègne le produit obtenu à l'étape (f) par une solution d'acétylacétonate de platine dans le toluène,

(h) on sèche le produit obtenu à l'étape (g),

(i) on calcine le produit obtenu à l'étape (h),

(j) on réduit sous courant d'hydrogène le produit obtenu à l'étape (i).

Une autre méthode avantageuse de préparation des catalyseurs peut être réalisée selon les étapes suivantes :

(a) on imprègne un support d'alumine avec une solution aqueuse de nitrate de lithium,

(b) on sèche le produit obtenu à l'étape (a),

(c) on calcine le produit obtenu à l'étape (b),

(d) on imprègne, avec une solution ammoniacale de chlorure de platine tétrammine, le produit obtenu à l'étape (c),

(e) on sèche le produit obtenu à l'étape (d),

(f) on calcine le produit sec obtenu à l'étape (e),

(g) on réduit le produit obtenu à l'étape (f) sous un courant d'hydrogène,

(h) on met en contact le produit obtenu à l'étape (g) avec un solvant hydrocarboné et avec ledit composé organique dudit métal M, par exemple en immergeant la masse dans un solvant hydrocarboné renfermant déjà le composé organique ou en immergeant la masse dans un solvant hydrocarboné et en injectant ensuite dans le mélange obtenu une solution du composé organique dudit métal M dans un solvant hydrocarboné et par exemple celui dans lequel ladite masse a été immergée,

(i) on réduit sous courant d'hydrogène le produit obtenu à l'étape (h).

Les exemples suivants illustrent l'invention sans en limiter la portée.

## EXEMPLE 1

On se propose de transformer du propane en présence d'un catalyseur à base de mélange d'une ziolithe MFI de rapport Si/Al = 45 et d'une alumine contenant du platine, un métal choisi dans le groupe constitué par l'étain, le germanium, l'indium et le plomb et du lithium et/ou du potassium.

. Préparation de la zéolithe MFI :

La zéolithe MFI est synthétisée en présence de structurant organique en utilisant une des recettes connues dans l'art antérieur (US-A-3.702.886). Cette zéolithe est transformée en une forme H par les traitements suivants :
  - calcination sous un mélange air-azote (10 % d'oxygène dans le mélange) à 550°C pendant 4 heures,
  - trois échanges dans $NH_4NO_3$ 5N à 100°C,
  - calcination sous air à 530°C pendant 5 heures sous un débit de 5 l/h/g.

Le rapport Si/Al de la zéolithe HMFI est égal à 45, son volume poreux mesuré par adsorption d'azote à 77K supérieur à 0,160 cm³/g.

. Préparation de l'alumine renfermant les métaux choisis :

Préparation d'une alumine A renfermant 0,30 % de platine en poids.

L'alumine A est préparée en ajoutant à 100 grammes d'alumine (de surface spécifique 240 m²/g et de volume poreux 0,58 cm³/g) 100 cm³ d'une solution d'acétyl acétonate de platine dans le toluène. La concentration en platine de cette solution est égal à 3 grammes par litre.

On laisse 6 heures en contact, on essore, on sèche 1 heure à 100-120°C puis on calcine 2 heures à 530°C. Puis on réduit sous courant d'hydrogène sec pendant 2 heures à 450°C.

De la même façon on prépare deux alumines A' et A'' renfermant respectivement 0,60 % et 1,1 % de platine, en poids.

. Préparation d'une alumine B (comparatif) renfermant en poids 0,30 % de platine et 0,5 % de lithium.

Le produit (B) (comparatif) est préparé en ajoutant à 100 grammes de support d'alumine précédent 100 cm³ d'une solution aqueuse de nitrate de lithium.

On laisse en contact 6 heures, on essore on sèche 1 heure à 100-120°C puis on abaisse 2 heures à 530°C.

Sur le produit calciné contenant le lithium on procède à l'imprégnation du platine de la même façon que le produit (A).

. Préparation d'une alumine C renfermant en poids 0,3 % de platine, 0,3 % d'étain et 0,5 % de lithium.

Après avoir fixé le lithium selon la même procédure que celle adoptée pour le produit (B), une solution acqueuse d'acétate d'étain est mise en contact avec le support d'alumine à raison de 100 cm³ de solution pour 100 grammes de support pendant 6 heures. Le solide obtenu est alors essoré et séché 1 heure à 100-120°C puis calciné à 530°C.

On procède alors sur le solide calciné contenant le lithium et l'étain à l'imprégnation du platine selon la même procédure que celle adoptée pour le produit A.

. Préparation d'une alumine D renfermant en poids 0,3 % de platine, 0,3 % de germanium et 0,5 % de lithium.

Le mode de préparation du produit D est strictement identique à celui décrit pour le produit C, si ce n'est que l'on utilise de l'oxalate de germanium en lieu et place de l'acétate d'étain.

. Préparation d'une alumine E renfermant en poids 0,3 % de platine, 0,3 % de plomb et 0,5 % de lithium.

Le mode de préparation du produit E est strictement identique à celui décrit pour le produit C, si ce n'est qu'en lieu et place de l'acétate d'étain on utilise de l'acétate de plomb.

. Préparation d'une alumine F renfermant en poids 0,3 % de platine, 0,3 % d'indium et 0,5 % de lithium.

Le mode de préparation du catalyseur F est identique à celui décrit pour le catalyseur C, si ce n'est qu'en lieu et place de l'acétate d'étain on utilise du nitrate d'indium.

. Préparation d'une alumine G renfermant en poids 0,3 % de platine, 0,3 % d'étain et 0,5 % de lithium.

Le produit B préparé ci-dessus est réduit sous courant d'hydrogène pendant deux heures à 450°C ; on immerge 100 grammes de ce catalyseur dans 300 cm³ de n-heptane. Ensuite on injecte, dans le n-heptane contenant le catalyseur, 3 grammes d'une solution de tétra n-butyl étain dans le n-heptane (à 10 % en étain). Le contact entre le catalyseur au platine et la solution de tétra n-butyl étain est maintenu pendant 6 heures à la température de reflux de l'heptane. La solution d'imprégnation est ensuite évacuée et l'on procède à 3 lavages par du n-heptane pur à la température de reflux du n-heptane. Le catalyseur est alors séché. Il peut ensuite subir soit une calcination sous air pendant 2 heures à 500°C suivie d'une réduction sous courant d'hydrogène à 450°C pendant deux heures avant d'être chargée dans le réacteur soit subir une réduction directe sous courant d'hydrogène à 450°C pendant deux heures avant d'être chargé dans le réacteur.

. Préparation d'une alumine H renfermant 0,30 % de platine et 0,30 % d'étain (en poids) (alumine comparative).

Le catalyseur H est préparé en ajoutant à 100 grammes d'alumine 100 cm³ d'une solution d'acétylacétonate de platine dans le toluène. La concentration de platine de cette solution est égale à 9 g/litre.

On laisse en contact 6 heures, on essore, on sèche une heure à 120°C puis on calcine 2 heures à 530°C sous air sec.

On procède alors à la fixation de l'étain selon le protocole décrit pour la préparation du produit C.

EP 0 458 674 B1

. Préparation d'un catalyseur I : on prépare un catalyseur semblable et préparé comme le catalyseur C mais renfermant également du germanium introduit dans la masse catalytique comme indiqué pour le catalyseur D. Dans le catalyseur I, il y a 0,15 % de germanium et 0,15 % d'étain.

EXEMPLE 2

On a testé les alumines préparées ci-dessus en mélange ou non avec la zéolithe MFI préparée également ci-dessus. La zéolithe a également été testée seule. Ces produits sont soumis à un test de transformation du propane dans les conditions suivantes :
- température :        450°C
- pression :        atmosphérique
- pph :        0,5 h⁻¹
- charge :        C₃H₈.
Il a, tout d'abord, été procédé un test des deux solides, zéolithe MFI et catalyseur C, seuls et en mélange. Résultats ci-dessous après 2 heures de réaction.

TABLEAU I

| Catalyseur | Conversion (% mole) | Sélectivité (% mole) | | | | |
|---|---|---|---|---|---|---|
| | | $CH_4$ | $C_2H_6$ | $C_2H_4$ | $C_3H_6$ | Aromatiques |
| Zéolithe MFI (comparatif) | 14 | 45 (26,6) | 15 (16,6) | 10 (10,3) | 30 (46,5) | 0 |
| Catalyseur C (Pt+Sn+Li/Alumine) | 20 | 5 (2) | 5 (3,7) | 0 | 90 (94,3) | 0 |
| Mélange ( 50 % zéolithe ( 50 % catalyseur C | 50 | 15 (6,0) | 30 (22,3) | 10 (6,9) | 25 (26,1) | 20 (38,7) |
| Mélange ( 25 % zéolithe ( 75 % catalyseur C | 25 | 5 (1,5) | 15 (8,6) | 5 (2,7) | 35 (27,9) | 40 (59,3) |
| Mélange ( 10 % zéolithe ( 90 % catalyseur C | 18 | 5 (1,7) | 10 (6,4) | 0 | 65 (58,5) | 20 (33,4) |

* NB : Entre parenthèses, on a indiqué les rendements pondéraux, basés uniquement sur $C_6H_6$ pour les aromatiques.

7

Alors que la zéolithe seule produit plus de 50 % de gaz légers ($CH_4$ et $C_2H_8$) et que le catalyseur C ne produit pratiquement que du propylène, l'association des deux solides permet d'augmenter sensiblement la sélectivité de production des aromatiques.

EXEMPLE 3 (comparatif)

Le tableau II donne les conversions et sélectivités obtenues avec la MFI seule et des mélanges de la MFI avec successivement le solide A qui renferme de l'alumine et 0,30 % de platine en poids, de solide A' où la teneur en platine passe à 0,6 % poids, le solide A" où la teneur pondérale en platine est 1,1 % et le solide H renfermant de l'alumine et en poids 0,3 % de platine et 0,3 % d'étain.

## TABLEAU II

| Catalyseur | Conversion (% moles) | Sélectivité (% moles) | | | | |
|---|---|---|---|---|---|---|
| | | $CH_4$ | $C_2H_6$ | $C_2H_4$ | $C_3H_6$ | Aromatiques |
| MFI | 14 | 45 | 15 | 10 | 30 | 0 |
| Mélange 25 % MFI + 75 % A | 15 | 35 | 30 | 5 | 20 | 10 |
| Mélange 25 % MFI + 75 % A' | 18 | 33 | 30 | 5 | 20 | 12 |
| Mélange 25 % MFI + 75 % A" | 17 | 34 | 30 | 5 | 20 | 11 |
| Mélange 25 % MFI + 75 % H | 20 | 7 | 18 | 5 | 40 | 30 |

EXEMPLE 4

25 % de zéolithe MFI (en poids) de rapport Si/Al = 45 préparée selon l'exemple 1 ont été mélangés à 75 % poids de catalyseur A (comparatif), B (comparatif) et C respectivement. Les résultats sont reportés dans le tableau III présenté ci-dessous.

TABLEAU III

| Catalyseur | Conversion (% mole) | Sélectivité (% mole) | | | | |
|---|---|---|---|---|---|---|
| | | $CH_4$ | $C_2H_6$ | $C_2H_4$ | $C_3H_8$ | Aromatiques |
| Zéolithe MFI + Catalyseur A Platine/alumine | 15 | 35 | 30 | 5 | 20 | 10 |
| Zéolithe MFI + Catalyseur B Platine+lithium/alumine | 30 | 35 | 40 | 5 | 10 | 10 |
| Zéolithe MFI + Catalyseur C Pt+Sn+Li/Alumine | 25 | 5 | 15 | 5 | 35 | 40 |

Les valeurs sont données après 2 heures de réaction. L'adjonction de catalyseur A n'améliore pas les performances au sens de l'activité et de la sélectivité. L'adjonction de catalyseur B améliore l'activité mais pas la sélectivité.

Le catalyseur C conforme à l'invention permet une bonne sélectivité en produits aromatiques.

EXEMPLE 5

Dans cet exemple nous utilisons une zéolithe MFI de rapport Si/Al = 36 synthétisée en absence de composé organique d'après un protocole de synthèse décrit dans "Studies in Surface Science and Catalysis", vol 33, 1987, p134. Après synthèse la zéolithe subit les traitements suivants :
- trois échanges dans $NH_4NO_3$ 10 à 100°C pendant 6 heures
- calcination sous air à 550°C 4 heures avec un débit de 3 l/h/g
- 25 % (poids) de zéolithe MFI de rapport Si/Al = 36, ont été mélangés à 75 % (poids) des catalyseurs C, D, E, F et G respectivement.

Les résultats obtenus dans les conditions opératoires précédentes sont rassemblés dans le tableau IV, présenté ci-dessous.

La nature de la MFI n'est pas critique puisque les mélanges MFI-catalyseur C dans les tableaux III et IV conduisent aux mêmes résultats.

TABLEAU IV

| Catalyseur | Conversion (% mole) | Sélectivité (% mole) | | | | |
|---|---|---|---|---|---|---|
| | | $CH_4$ | $C_2H_6$ | $C_2H_4$ | $C_3H_8$ | Aromatiques |
| Zéolithe MFI + Catalyseur C (Pt+Sn+Li) | 25 | 5 | 15 | 5 | 35 | 40 |
| Zéolithe MFI + Catalyseur D (Pt+Ge+Li) | 23 | 6 | 14 | 5 | 37 | 38 |
| Zéolithe MFI + Catalyseur E (Pt+Pb+Li) | 24 | 7 | 14 | 6 | 34 | 39 |
| Zéolithe MFI + Catalyseur F (Pt+In+Li) | 22 | 7 | 13 | 5 | 38 | 37 |
| Zéolithe MFI + Catalyseur G (Pt+Sn+Li) | 28 | 4 | 13 | 5 | 35 | 43 |
| Zéolithe MFI + Catalyseur I (Pt+Sn+Ge+Li) | 29 | 4 | 12 | 5 | 34 | 45 |

**Revendications**

1. Catalyseur renfermant (a) une zéolithe MFI, (b) une matrice renfermant au moins un métal noble de la famille du platine, au moins un métal additionnel choisi dans le groupe constitué par l'étain, le germanium, le plomb et l'indium et 0,01 à 2 % d'au moins un métal alcalin ou alcalino-terreux.

2. Catalyseur selon la revendication 1 renfermant en poids :
   (a) 1 à 99 % de zéolithe MFI
   (b) 99 à 1 % d'une matrice renfermant 0,01 à 2 % d'un métal de la famille du platine, 0,005 à 0,60 % de métal additionnel lorsque celui-ci est l'étain ou 0,005 à 0,70 % de métal additionnel lorsque celui-ci est le germanium, le plomb ou l'indium, et 0,01 à 2 % d'au moins un métal alcalin ou alcalino-terreux.

3. Catalyseur selon l'une des revendications 1 et 2 dans lequel la matrice est l'alumine.

4. Catalyseur selon l'une des revendications 1 à 3 dans lequel la matrice contient en poids 0,1 à 0,5 % d'au moins un métal noble de la famille du platine, 0,01 à 0,5 % d'étain ou 0,01 à 0,6 % de germanium, d'étain ou de plomb et 0,1 à 0,6 % d'au moins un métal alcalin ou alcalino-terreux.

5. Catalyseur selon l'une des revendications 1 à 4 renfermant à titre de dits métaux additionnels de l'étain et un métal choisi dans le groupe constitué par le germanium et le plomb, la teneur totale en métaux additionnels étant comprise entre 0,02 et 1,20 %.

6. Catalyseur selon l'une des revendications 1 à 5 renfermant une matrice d'alumine, du platine, au moins un métal additionnel dont l'étain et du lithium ou du potassium.

7. Utilisation du catalyseur selon l'une des revendications 1 à 6 dans les procédés d'aromatisation d'hydrocarbures renfermant 2 à 4 atomes de carbone par molécule.


**Patentansprüche**

1. Katalysator, umfassend (a) einen MFI-Zeolith, (b) eine Matrix, die wenigstens ein Edelmetall der Familie des Platins, wenigstens ein Zusatzmetall, das gewählt ist aus der Gruppe bestehend aus Zinn, Germanium, Blei und Indium und aus 0,01 bis 2% wenigstens eines Alkalimetalls oder Erdalkalimetalls umfasst.

2. Katalysator nach Anspruch 1, umfassend in Gewichtsprozent:
   (a) 1 bis 99% MFI-Zeolith
   (b) 99 bis 1% einer Matrix, die 0,01 bis 2% eines Metalls der Familie des Platins, 0,005 bis 0,60% Zusatzmetall, wenn dieses Zinn ist oder 0,005 bis 0,70% Zusatzmetall, wenn dieses Germanium, Blei oder Indium ist und 0,01 bis 2% wenigstens eines Alkalimetalls oder Erdalkalimetalls umfasst.

3. Katalysator nach einem der Ansprüche 1 und 2, bei dem die Matrix Aluminiumoxyd ist.

4. Katalysator nach einem der Ansprüche 1 bis 3, bei dem die Matrix in Gewichtsprozent enthält: 0,1 bis 0,5% wenigstens eines Edelmetalls der Familie des Platins, 0,01 bis 0,5% Zinn oder 0,01 bis 0,6% Germanium, Zinn oder Blei und 0,1 bis 0,6% wenigstens eines Alkalimetalls oder Erdalkalimitalls.

5. Katalysator nach einem der Ansprüche 1 bis 4, der als solche Zusatzmetalle Zinn und ein Metall umfasst, das gewählt ist aus der Gruppe, die gebildet wird aus Germanium und Blei, wobei der Gesamtgehalt an Zusatzmetallen zwischen 0,02 und 1,20% liegt.

6. Katalysator nach einem der Ansprüche 1 bis 5, umfassend eine Matrix aus Aluminiumoxyd, Platin, wenigstens ein Zusatzmetall, hier Zinn und Lizium oder Kalium.

7. Verwendung des Katalysators nach einem der Ansprüche 1 bis 6 im Verfahren zur Aromatisierung von Kohlenwasserstoffen, die 2 bis 4 Kohlenstoffatome pro Molekül umfassen.


**Claims**

1. Catalyst containing (a) a MFI zeolite, (b) a matrix containing at least one noble metal from the platinum group, at least one additional metal chosen from within the group constituted by tin, germanium, lead and indium and 0.01 to 2% of at least one alkali metal or alkaline earth metal.

2. Catalyst according to claim 1, containing by weight:
   (a) 1 to 99% MFI zeolite and

(b) 99 to 1% of a matrix containing 0.01 to 2% of a metal from the platinum group, 0.005 to 0.60% of an additional metal when the latter is tin or 0.005 to 0.70% of additional metal when the latter is germanium, lead or indium and 0.01 to 2% of at least one alkali metal or alkaline earth metal.

3. Catalyst according to one of the claims 1 and 2, wherein the matrix is alumina.

4. Catalyst according to any one of the claims 1 to 3, wherein the matrix contains, by weight, 0.1 to 0.5% of at least one noble metal from the platinum group, 0.01 to 0.5% tin or 0.01 to 0.6% germanium, tin or lead and 0.1 to 0.6% of at least one alkali metal or alkaline earth metal.

5. Catalyst according to any one of the claims 1 to 4 containing as said additional metals tin and a metal chosen from the group constituted by germanium and lead, the total additional metal content being between 0.02 and 1.20%.

6. Catalyst according to any one of the claims 1 to 5 containing a matrix of alumina, platinum, at least one additional metal, including tin and lithium or potassium.

7. Use of the catalyst according to any one of the claims 1 to 6 in processes for the aromatization of hydrocarbons containing 2 to 4 carbon atoms per molecule.